# EUROPEAN PATENT APPLICATION

(11) **EP 3 348 563 A1**
(43) Date of publication of application: **18.07.2018**
(21) Application number: 17151459.9
(22) Date of filing: 13.01.2017
(51) Int. Cl.: C07F 17/00, C08F 4/64, C08G 64/30, C08L 69/00, C07C 68/06

(54) **CATALYST FOR CONVERSION OF DIALKYL CARBONATE TO DIARYL CARBONATE AND METHOD OF USE**

(71) Applicant: SABIC Global Technologies B.V., 4612 PX Bergen op Zoom (NL)
(72) Inventor: Basale, Rajshkhar, Madhukar, 560037 Karnataka Banglore (IN)
(74) Representative: Sabic Intellectual Property Group

(57) **Abstract**

A catalyst comprising a compound of the formula

[(R)ₘCp]ₙM(OAr)₂L₂₋ₙ

wherein each Cp is cyclopentadienyl; each R is independently the same or different, and is a substituted or unsubstituted C₁₋₆ alkyl group; M is Ti, Zr, or Hf, each Ar is independently the same or different, and is a substituted or unsubstituted C₆₋₂₀ aromatic hydrocarbon group; each L is independently the same or different, and is a halogen or a substituted or unsubstituted C₁₋₆ alkoxide group; m is 0 to 5, and n is 1 or 2.

## Description

### Background

Polycarbonate (PC) is an important engineering thermoplastic with excellent mechanical, optical, electrical, and heat resistance properties. An advantageous route for the manufacture of polycarbonates is based on the melt transesterification of a diaryl carbonate (e.g., diphenyl carbonate (DPC)) with an aromatic dihydroxy compound, in particular a bisphenol such as bisphenol A (BPA). It is also possible to obtain polycarbonates by reacting an activated diaryl carbonate with an aromatic dihydroxy compound in an interfacial process.

In the commercial methods for the production of diaryl carbonates such as DPC, a dialkyl carbonate (e.g., dimethyl carbonate) is reacted with an aromatic hydroxyl compound (e.g., phenol) using a carbonate interchange reaction. However, this reaction can be slow and equilibrium limited, mainly due to the weak nucleophilicity of aromatic hydroxyl compounds, and thus requires the input of energy. A Lewis acid catalyst can be used for this reaction, and titanium catalysts are commonly utilized.

The present titanium catalysts can be sensitive to water and readily hydrolyze to titanium dioxide (TiO₂), which can limit the recovery of the catalyst after the reaction is complete. Titanium dioxide can also cause erosion of the reactors and other plant equipment. Thus, there remains a need for the development of new catalysts having selective catalytic activity for the manufacture of diaryl carbonates. It would be a further advantage if the catalysts were stable in in water.

### SUMMARY

The above-described and other deficiencies of the art are met by a catalyst including a compound of the formula [(R)ₘCp]ₙM(OAr)₂L₂₋ₙ, wherein each Cp is cyclopentadienyl; each R is independently the same or different, and is a substituted or unsubstituted C₁₋₆ alkyl group; M is Ti, Zr, or Hf, each Ar is independently the same or different, and is a substituted or unsubstituted C₆₋₂₀ aromatic hydrocarbon group; each L is independently the same or different, and is a halogen or a substituted or unsubstituted C₁₋₆ alkoxide group; m is 0 to 5, and n is 1 or 2.

A method for the synthesis of the catalyst includes reacting a metallocene halide of the formula [(R)ₘCp]ₙMX₄₋ₙ, wherein X is F, Cl, Br, or I, with an aromatic hydroxy compound of the formula ArOH to obtain the catalyst.

A method for the synthesis of a diaryl carbonate includes reacting, in the presence of the catalyst of any one or more of claims 1 to 3, or made by the methods of any one or more of claims 4 to 8 a dialkyl carbonate of the formula O=C(OZ)₂ wherein each Z is independently the same or different, and is a C₁₋₄ alkyl group, with an aromatic aromatic alcohol of the formula 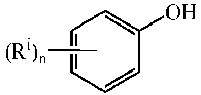 wherein n is 0 to 2 and each Rⁱ is independently the same or different and is nitro, halo, (C₁₋₁₈hydrocarbyl)carbonyl, or (C₁₋₃alkoxy)carbonyl to provide the diaryl carbonate of the formula

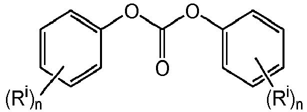

IA method for the manufacture of a polycarbonate includes manufacturing the diaryl carbonate by the above method; and reacting the diaryl carbonate with a dihydroxy aromatic compound under conditions effective to provide the polycarbonate.

A polycarbonate composition is provided and is manufactured by the methods described herein.

The above described and other features are exemplified by the following drawings, detailed description, examples, and claims.

### BRIEF DESCRIPTION OF THE DRAWINGS

The following figures are exemplary embodiments wherein the like elements are numbered alike.
FIG. 1 is a graph of intensity (arbitrary units, au) versus chemical shift (parts per million, ppm) showing a proton nuclear magnetic resonance (¹H NMR) spectrum of titanocene dichloride according to an exemplary embodiment;
FIG. 2 is a graph of intensity (au) versus chemical shift (ppm) showing a ¹H NMR spectrum of diphenoxy titanocene according to an exemplary embodiment;

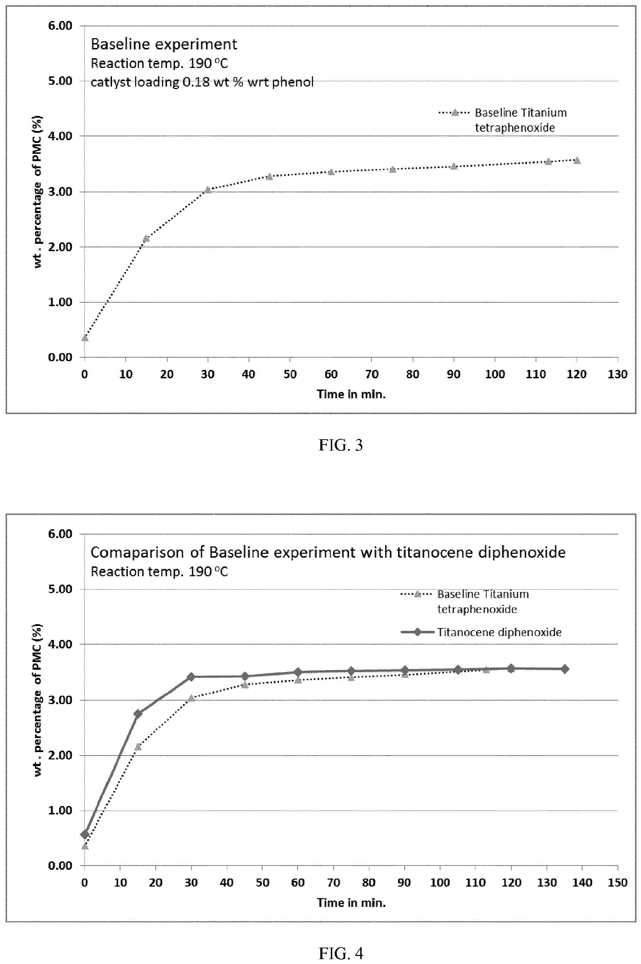

FIG. 3 is a graph of weight percent (wt%) versus time (minutes) for a titanium tetraphenoxide catalyst according to an exemplary embodiment; and
FIG. 4 shows a graph of weight percent (wt%) versus time (minutes) for a titanocene diphenoxide catalyst according to an exemplary embodiment.

### DETAILED DESCRIPTION

The inventors have discovered novel catalysts having desirable transesterification selectivity for use in the manufacture diarylcarbonates from dialkyl carbonates. The diarylcarbonates can subsequently be reacted with a dihydroxy aromatic compound under conditions effective to provide a polycarbonate.

The catalyst is a compound of formula (1)

[(R)ₘCp]ₙM(OAr)₂L₂₋ₙ (1)

wherein each Cp is cyclopentadienyl; each R is independently the same or different, and is a substituted or unsubstituted C₁-₆ alkyl group; M is titanium (Ti), (Zr), or hafnium (Hf); each Ar is independently the same or different, and is a substituted or unsubstituted C₆₋₂₀ aromatic hydrocarbon group; each L is independently the same or different, and is a halogen or a substituted or unsubstituted C₁₋₆ alkoxide group; m is 0 to 5, and n is 1 or 2.

In a specific embodiment in formula (1), each R is independently the same or different, and is an unsubstituted C₁₋₆ alkyl group; M is Ti, Zr, or Hf; each Ar is independently the same or different, and is a substituted or unsubstituted phenyl group; each L is independently the same or different, and is a halogen or an unsubstituted C₁₋₆ alkoxide group; m is 0 to 3, and n is 1 or 2.

In another specific embodiment in formula (1), each R is the same and is an unsubstituted C₁₋₃ alkyl group; M is Ti, Zr, or Hf; each Ar is the same and is a phenyl group; each L is independently the same or different, and is a halogen or an unsubstituted C₁₋₃ alkoxide group; m is 0 to 2, and n is 1 or 2.

In any of the foregoing embodiments, M is preferably Ti. In some embodiments, the catalyst is of formula (1a) 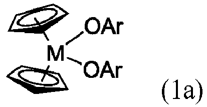 wherein M is Ti, Zr, or Hf, and Ar is as defined in any of the embodiments above, but is preferably unsubstituted phenyl. In a specific embodiment M is Ti and Ar is unsubstituted phenyl.

The catalyst of formulas (1) or (1a) can be prepared by reacting a metallocene halide of the formula

[(R)ₘCₚ]ₙMX₄₋ₙ (2)

wherein R, Cp, M, m, and n are as described in formulas (1) and (1a) and X is F, Cl, Br, or I, with an aromatic hydroxy compound of formula (3)

ArOH (3)

wherein Ar is as defined in formulas (1) and (1a).

In a specific embodiment, the metallocene halide is of formula (2a) 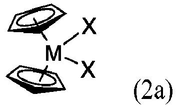 wherein M is Ti, Zr, or Hf, preferably Ti; and X is Cl or Br, or I.

The catalyst can be readily prepared by combining the metallocene (2) and the hydroxy compound (3) in the presence of a base and a solvent.

The base can be an organic amine, for example pyridine, N,N'-dimethylamino pyridine, N-methylpyrrolidone, dimethyl amine, trimethyl amine, triethyl amine, ethyldiisopropyl amine, N-methylimidazole, N,N-dimethylaniline, or the like, or a combination comprising at least one of the foregoing. The base can be present in an amount of 1 to 5 moles based on the moles of metallocene halide, for example 2 to 3 moles.

The solvent can be a protic solvent such as methanol, ethanol, isopropanol, or the like.

The catalyst can be prepared using a molar ratio of the hydroxy compound (3) to metallocene (2) that is 1:1 to 6:1, preferably 2:1 to 4:1, more preferably 2:1 to 3:1. The reaction can be performed, for example, at a temperature of 25 to 200 °C, preferably 25 to 180 °C, more preferably 25 to 150 °C, for a time effective to provide the catalyst, for example 10 minutes to 6 hours, or 20 minutes to 4 hours, or 30 minutes to 3 hours.

The catalyst is effective in the transesterification of a dialkyl carbonate of formula (4)

O=(OZ)₂ (4)

each Z is independently the same or different, and is a branched or unbranched C₁₋₄ alkyl group, with an aromatic alcohol of formula (5) 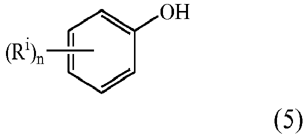 to provide the diaryl carbonate of formula (6) 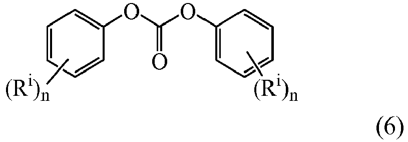 wherein in formulas (5) and (6) n is 0 to 2 and each Rⁱ is independently the same or different and is an activating group. The partial transesterification product (7) and diether (8) 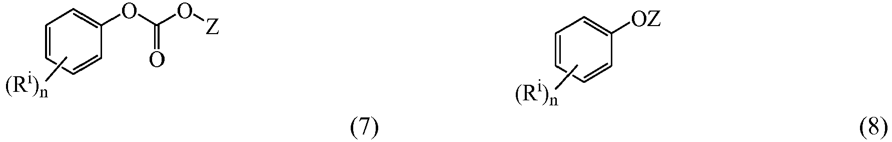 can also be formed, wherein Z, Rⁱ, and n are as described in formulas (4) and (5).

In an embodiment, each Z is the same, and is an unsubstituted C₁₋₆ alkyl group, preferably a methyl group.

Activating groups Rⁱ include nitro, halo (specifically chloro), (C₁₋₁₈hydrocarbyl)carbonyl, and (C₁₋₃alkoxy)carbonyl, and are present at the 2- or 4-positions of the phenyl, or both. In an embodiment, the the diaryl carbonate (8) is diphenyl carbonate, bis(4-nitrophenyl)carbonate, bis(2-chlorophenyl)carbonate, bis(4-chlorophenyl)carbonate, bis(methyl salicylate)carbonate, bis(4-methylcarboxyphenyl) carbonate, bis(2-acetylphenyl) carboxylate, or bis(4-acetylphenyl) carboxylate.

In an embodiment, dimethyl carbonate (DMC) is transesterified with phenol in the presence of the catalyst. The products of the reaction include diphenyl carbonate (DPC), methylphenyl carbonate (MPC), and anisole.

Conditions for transesterification of the dialkyl carbonate and the aromatic hydroxyl compound in the presence of catalyst (1) or (1a) are generally known, being described, for example, in U.S. Pat. No. 5,344,954 and U.S. Pat. No. 5,747,609. The reaction temperature can be varied depending on the dialkyl carbonate used and diaryl carbonate produced. In general, the reaction temperature can be 50 to 350°C., or 120 to 280°C. The reaction pressure is not critical and can be selected within wide ranges. It is feasible to conduct the reaction at subatmospheric, atmospheric, and super-atmospheric pressure. The reaction pressure can be 0.01 to 100 bar (1 kPa to 10 MPa), preferably 1 to 50 bar. The reaction time can be 2 minutes to 50 hours, or 5 minutes to 25 hours, or 10 minutes to 12.5 hours. The diaryl carbonate can be isolated by known methods, or used directly.

The transesterification selectivity of the catalyst of formulas (1) or (1a) can be substantially the same as a transesterification selectivity of a comparable reaction in the presence of a catalyst having the formula M'(OAr')₄, wherein M' is Ti, Zr, or Hf, and is the same as M of the catalyst, and Ar' is a substituted or unsubstituted C₆₋₂₀ aromatic hydrocarbon group and is the same as Ar of the catalyst.

The transesterification selectivity of the catalyst can be greater than 98%, preferably greater than 98.5%, more preferably greater than 99%, even more preferably greater than 99.5%.

The diaryl carbonates are of special utility in the manufacture of polycarbonates. "Polycarbonate" as used herein means a polymer or copolymer having repeating structural carbonate units of formula (1) 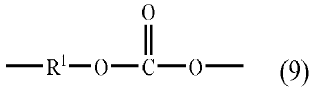 wherein at least 60 percent of the total number of R¹ groups are aromatic, or each R¹ contains at least one C₆₋₃₀ aromatic group. Polycarbonates and their methods of manufacture are known in the art, being described, for example, in WO2013/027165, WO 2013/175448 A1, US 2014/0295363, and WO 2014/072923. Polycarbonates are generally manufactured from dihydroxy reactants such resorcinol, 2,2-bis(4-hydroxyphenyl) propane ("bisphenol A" or "BPA"), 3,3-bis(4-hydroxyphenyl) phthalimidine, 2-phenyl-3,3'-bis(4-hydroxyphenyl) phthalimidine (also known as N-phenyl phenolphthalein bisphenol, "PPPBP", or 3,3-bis(4-hydroxyphenyl)-2-phenylisoindolin-1-one), 1,1-bis(4-hydroxy-3-methylphenyl)cyclohexane, and 1,1-bis(4-hydroxyphenyl)-3,3,5-trimethylcyclohexane (isophorone bisphenol), or a combination comprising at least one of the foregoing. In a specific embodiment, the polycarbonate is a homopolymer derived from BPA; or a copolymer derived from BPA and another bisphenol or dihydroxy aromatic compound such as resorcinol.

In a preferred embodiment, melt processes can be used to make the polycarbonates, by co-reacting, in a molten state, a dihydroxy reactant as described above and a diaryl carbonate ester as described above in the presence of a transesterification catalyst. Conditions for melt process are described, for example, in WO2013/027165 and the references cited therein. Catalysts used in the melt polymerization can include an alpha catalyst and a beta catalyst. Alpha catalysts can comprise a source of alkali or alkaline earth ions and are typically more thermally stable and less volatile than beta catalysts. Beta catalysts are typically volatile and degrade at elevated temperatures, and can comprise a tranesterification catalyst of the formula (R³)₄Q⁺X as described above. Beta catalysts are therefore preferred for use at early low-temperature polymerization stages. The alpha catalyst can be used in an amount sufficient to provide 1 x 10⁻² to 1 x 10⁻⁸ moles, specifically, 1 x 10⁻⁴ to 1 x 10⁻⁷ moles of metal per mole of the dihydroxy compounds used. The amount of beta catalyst (e.g., organic ammonium or phosphonium salts) can be 1 x 10⁻² to 1 x 10⁻⁵, specifically 1 x 10⁻³ to 1 x 10⁻⁴ moles per total mole of the dihydroxy compounds in the reaction mixture.

Quenching of the transesterification catalysts and any reactive catalysts residues with an acidic compound after polymerization is completed can also be useful in some melt polymerization processes. Among the many quenchers that can be used are alkyl sulfonic esters of the formula R⁸SO₃R⁹ wherein R⁸ is hydrogen, C₁-C₁₂ alkyl, C₆-C₁₈ aryl, or C₇-C₁₉ alkylaryl, and R⁹ is C₁-C₁₂ alkyl, C₆-C₁₈ aryl, or C₇-C₁₉ alkylaryl (e.g., benzenesulfonate, p-toluenesulfonate, methylbenzene sulfonate, ethylbenzene sulfonate, n-butyl benzenesulfonate, octyl benzenesulfonate and phenyl benzenesulfonate, methyl p-toluenesulfonate, ethyl p-toluenesulfonate, n-butyl p-toluene sulfonate, octyl p-toluenesulfonate and phenyl p-toluenesulfonate, in particular alkyl tosylates such as n-butyl tosylate).

Alternatively, where the diaryl carbonate is an activated diaryl carbonate, an interfacial process can be used. Although the reaction conditions for interfacial polymerization can vary, an exemplary process generally involves dissolving or dispersing a dihydroxy reactant in aqueous NaOH or KOH, adding the resulting mixture to a water-immiscible solvent, and contacting the reactants with the diaryl carbonate in the presence of a catalyst such as a tertiary amine or a phase transfer catalyst, under controlled pH conditions, e.g., 8 to 10. The water-immiscible solvent can be, for example, methylene chloride, 1,2-dichloroethane, chlorobenzene, toluene, and the like. Among tertiary amines that can be used as catalysts in interfacial polymerization are aliphatic tertiary amines such as triethylamine and tributylamine, cycloaliphatic tertiary amines such as N,N-diethyl-cyclohexylamine, and aromatic tertiary amines such as N,N-dimethylaniline. Among the phase transfer catalysts that can be used are catalysts of the formula (R³)₄Q⁺X, wherein each R³ is the same or different, and is a C₁₋₁₀ alkyl; Q is a nitrogen or phosphorus atom; and X is a halogen atom or a C₁₋₈ alkoxy or C₆₋₁₈ aryloxy. Exemplary phase transfer catalysts include (CH₃(CH₂)₃)₄NX, (CH₃(CH₂)₃)₄PX, (CH₃(CH₂)₅)₄NX, (CH₃(CH₂)₆)₄NX, (CH₃(CH₂)₄)₄NX, CH₃(CH₃(CH₂)₃)₃NX, and CH₃(CH₃(CH₂)₂)₃NX, wherein X is Cl⁻, Br⁻, a C₁₋₈ alkoxy or a C₆₋₁₈ aryloxy. An effective amount of a phase transfer catalyst can be 0.1 to 10 wt%, or 0.5 to 2 wt%, each based on the weight of dihydroxy compound in the phosgenation mixture.

The diaryl carbonate and dihydroxy reactant can be introduced to, for example, a mixer or a reactor in a molar ratio of 2:1 to 1:2, specifically in a molar ratio of 1.5:1 to 1:1.5, more specifically in a molar ratio of 1.05:1 to 1:1.05, even more specifically in a molar ratio of 1:1. In an embodiment, the molar ratio of the diaryl carbonate to the dihydroxy compound when expressed to three decimal places is 0.996 or less, or 0.962 to 0.996, or 0.968 to 0.996, or 0.971 to 0.994

An end-capping agent as is known in the art can be included during polymerization to provide end groups. The end-capping agent (and thus end groups) are selected based on the desired properties of the polycarbonates. Exemplary end-capping agents are exemplified by monocyclic phenols such as phenol and C₁-C₂₂ alkyl-substituted phenols such as p-cumyl-phenol, resorcinol monobenzoate, and p-and tertiary-butyl phenol, monoethers of diphenols, such as p-methoxyphenol, and alkyl-substituted phenols with branched chain alkyl substituents having 8 to 9 carbon atoms, 4-substituted-2-hydroxybenzophenones and their derivatives, aryl salicylates, monoesters of diphenols such as resorcinol monobenzoate, 2-(2-hydroxyaryl)-benzotriazoles and their derivatives, 2-(2-hydroxyaryl)-1,3,5-triazines and their derivatives, mono-carboxylic acid chlorides such as benzoyl chloride, C₁-C₂₂ alkyl-substituted benzoyl chloride, toluoyl chloride, bromobenzoyl chloride, cinnamoyl chloride, and 4-nadimidobenzoyl chloride, polycyclic, mono-carboxylic acid chlorides such as trimellitic anhydride chloride, and naphthoyl chloride, functionalized chlorides of aliphatic monocarboxylic acids, such as acryloyl chloride and methacryoyl chloride, and mono-chloroformates such as phenyl chloroformate, alkyl-substituted phenyl chloroformates, p-cumyl phenyl chloroformate, and toluene chloroformate. Combinations of different end groups can be used.

Branched polycarbonate blocks can be prepared by adding a branching agent during polymerization. These branching agents include polyfunctional organic compounds containing at least three functional groups selected from hydroxyl, carboxyl, carboxylic anhydride, haloformyl, and mixtures of the foregoing functional groups. Specific examples include trimellitic acid, trimellitic anhydride, trimellitic trichloride, tris-p-hydroxyphenylethane, isatin-bis-phenol, tris-phenol TC (1,3,5-tris((p-hydroxyphenyl)isopropyl)benzene), tris-phenol PA (4(4(1,1-bis(p-hydroxyphenyl)-ethyl) alpha, alpha-dimethyl benzyl)phenol), 4-chloroformyl phthalic anhydride, trimesic acid, and benzophenone tetracarboxylic acid. The branching agents can be added at a level of 0.05 to 2.0 wt.%. Combinations comprising linear polycarbonates and branched polycarbonates can be used.

Each of the catalyst synthesis, transesterification, and polymerization can be carried out in a batch, semi-batch, or continuous mode separately or together. In an embodiment, the catalyst is synthesized in batch or sem-batch mode, and the transesterification, polymerization, or both are carried out in continuous mode.

The polycarbonates can be used in any application, and be further processed to include additives as is known in the art. The polycarbonate compositions can be molded into useful shaped articles by a variety of methods, such as injection molding, extrusion, rotational molding, blow molding, and thermoforming. Some example of articles include computer and business machine housings such as housings for monitors, handheld electronic device housings such as housings for cell phones, electrical connectors, and components of lighting fixtures, ornaments, home appliances, roofs, greenhouses, sun rooms, swimming pool enclosures, and the like.

This disclosure is further illustrated by the following examples, which are nonlimiting.

### EXAMPLES

The following components are used in the examples. Unless specifically indicated otherwise, the amount of each component is in weight percent in the following examples, based on the total weight of the composition.

**Materials.**

| **Component** | **Source** |
|---|---|
| Titanocene dichloride | Sigma-Aldrich |
| Dimethyl carbonate (DMC) | Sigma-Aldrich |
| Phenol | Sigma-Aldrich |
| Titanium tetraphenoxide | SABIC |
| Pyridine | Sigma-Aldrich |
| Chloroform-d | Cambridge Isotope Laboratories |

All solvents were used as received.

### Analytical methods.

Proton nuclear magnetic resonance (¹H NMR) spectroscopy characterization was performed on a Varian Unity 400 at 399.98 MHz in deuterated chloroform.

Gas chromatography (GC) was performed on an Agilent GC 6890B equipped with and autosampler and a flame ionization detector (FID).

### Preparation of catalyst Titanocene diphenoxide.

Titanocene dichloride (1 mole) was reacted with phenol (2 moles) in the presence of pyridine (2 moles) as a base at temperature 80 °C for 3 hrs. The reaction mass was then stirred in the presence of ethanol as solvent for 30 minutes (min), and the ethanol layer was separated using a separatory funnel. The traces of ethanol were removed by using a rotary evaporator at 50 °C under vacuum.

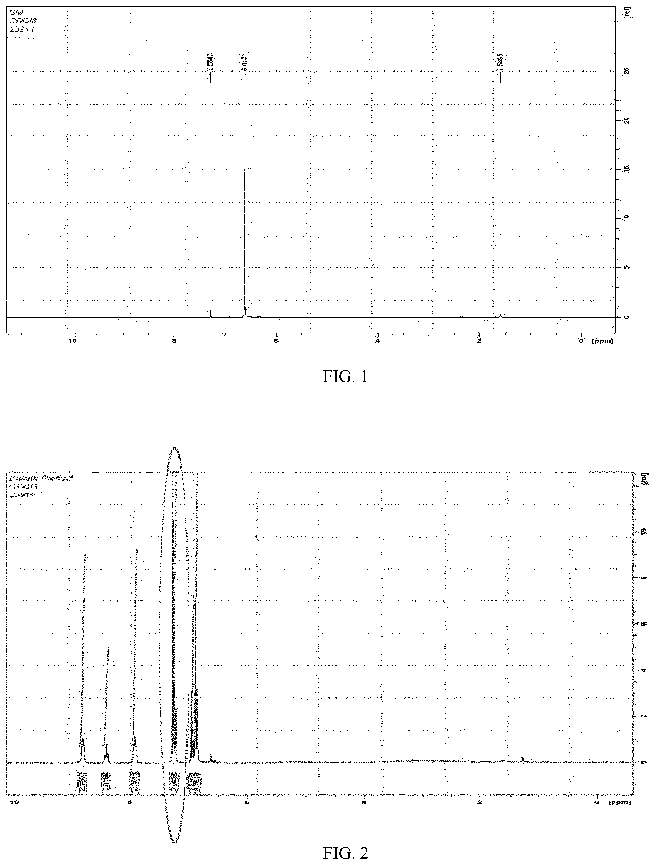

The catalyst thus obtained (titanocene diphenoxide) was analyzed by ¹H NMR (FIGS. 1 and 2), which showed the presence of aromatic protons from the phenoxide groups (FIG. 2), indicating the conversion of starting titanocene dichloride into the product catalyst titanocene diphenoxide.

### Catalyst Activity

### Example 1.

The titanocene diphenoxide catalyst was prepared as described above. Phenol, dimethyl carbonate (DMC), and the titanocene diphenoxide catalyst were charged into a 300 milliliter (mL) Parr reactor. The reactor was closed and heated slowly to 190 °C. Once the reaction mixture temperature reached to 190°C, samples were periodically withdrawn and monitored via GC analysis to determine the amount (wt %) of phenol, DMC, methylphenyl carbonate (MPC), and methanol. Reaction kinetics were then analyzed over the course of the reaction.

### Example 2.

In order to determine selectivity of the PMC product, reactions were performed at a higher conversion (%) of phenol. A stainless steel pressure reactor (200 mL), jacketed with heating coils and attached to a column packed with molecular sieves, was used. The column outlet was connected to a catch pot via a back pressure regulator and a bypass line. A digital temperature sensor and a pressure gauge were used to monitor the temperature and pressure.

Phenol and DMC (molar ratio 2:1) and the titanocene diphenoxide catalyst (0.18 wt%) were charged into the reactor. Initially, the reactor was pressurized to 5 bar under nitrogen gas. The pressure in the reactor was adjusted to 4 bar with a back pressure regulator. The temperature of the reactor was slowly increased by the heating coils and set to 230 °C. Reaction mixture samples were withdrawn at regular time intervals for GC analysis. After completion of the reaction, heating was stopped and reaction mixture was removed via a bottom valve.

The conversion (%) and selectivity (%) thus obtained from the reaction is shown in Table 1.

**Table 1.**

| **PMC + DPC (%)** | **Anisole (%)** | **Selectivity (%)** |
|---|---|---|
| 5 | 0.02 | 99.60 |
| 15 | 0.14 | 99.05 |
| 26 | 0.38 | 98.55 |
| 30 | 0.54 | 98.24 |

### Example 3.

As a comparative example, phenol, DMC (molar ratio 2:1), and a titanocene tetraphenoxide catalyst (0.18 wt%) were charged into a reactor. The reaction conditions were the same as in Example 2, and data was obtained via GC analysis. The conversion (%) and selectivity (%) of the titanocene tetraphenoxide catalyst for this same reaction is shown in Table 2.

**Table 2.**

| **PMC + DPC (%)** | **Anisole (%)** | **Selectivity (%)** |
|---|---|---|
| 4.1 | 0.03 | 99.50 |
| 12 | 0.11 | 99.11 |
| 25 | 0.39 | 98.45 |
| 34 | 0.61 | 98.20 |

As can be seen from the data, the selectivity of the titanocene diphenoxide catalyst in Example 2 is comparable to the selectivity of the titanocene tetraphenoxide catalyst in Example 3. Additionally, the selectivity remained comparable at different conversion levels.

In summary, titanocene diphenoxide showed better activity and comparable selectivity with titanium tetraphenoxide. In addition, titanocene dichloride-based catalysts, such as the titanocene diphenoxide catalyst, have the added benefit of stability in water and air.

This disclosure further encompasses the following embodiments.
Embodiment 1. A catalyst including a compound of the formula [(R)ₘCp]ₙM(OAr)₂L₂₋ₙ, wherein each Cp is cyclopentadienyl; each R is independently the same or different, and is a substituted or unsubstituted C₁₋₆ alkyl group; M is Ti, Zr, or Hf, each Ar is independently the same or different, and is a substituted or unsubstituted C₆₋₂₀ aromatic hydrocarbon group; each L is independently the same or different, and is a halogen or a substituted or unsubstituted C₁₋₆ alkoxide group; m is 0 to 5, and n is 1 or 2.
Embodiment 2. The catalyst of embodiment 1, wherein the compound comprises the formula 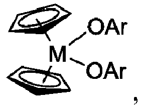 wherein M is Ti, Zr, or Hf.
Embodiment 3. The catalyst of embodiment 2 or 3, wherein M is Ti.
Embodiment 4. A method for the synthesis of the catalyst of any one or more of embodiments 1 to 3 includes reacting a metallocene halide of the formula [(R)ₘCp]ₙMX₄₋ₙ, wherein X is F, Cl, Br, or I, with an aromatic hydroxy compound of the formula ArOH to obtain the catalyst.
Embodiment 5. The method of embodiment 4, wherein the metallocene has the formula 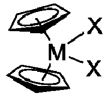
Embodiment 6. The method of embodiment 4 or 5, wherein M is Ti and X is Cl.
Embodiment 7: The method of any one or more of embodiments 4 to 6, wherein the reacting comprises combining the metallocene, the phenol, and a solvent.
Embodiment 8. The method of embodiment 7, wherein a molar ratio of the phenol to the metallocene is 1:1 to 6:1, preferably 2:1 to 4:1, more preferably 2:1 to 3:1; the solvent is pyridine, N,N'-dimethylamino pyridine, N-methylpyrrolidone, dimethyl amine, trimethyl amine, triethyl amine, ethyldiisopropyl amine, dimethylformamide, N-methylformamide, N-methylimidazole, N,N-dimethylaniline, pyridine, ammonia, or a combination comprising at least one of the foregoing; and the reacting is at a temperature of 25 °C to 2 °C, preferably 25 °C to 180 °C, more preferably 25 °C to 150 °C, for a time of 10 minutes to 6 hours, preferably 20 minutes to 4 hours, more preferably 30 minutes to 3 hours.
Embodiment 9. A method for the synthesis of a diaryl carbonate includes reacting, in the presence of the catalyst of any one or more of embodiments 1 to 3, or made by the methods of any one or more of embodiments 4 to 8 a dialkyl carbonate of the formula O=C(OZ)₂ wherein each Z is independently the same or different, and is a C₁₋₄ alkyl group, with an aromatic aromatic alcohol of the formula 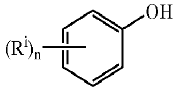 wherein n is 0 to 2 and each Rⁱ is independently the same or different and is nitro, halo, (C₁₋₁₈hydrocarbyl)carbonyl, or (C₁₋₃alkoxy)carbonyl to provide the diaryl carbonate of the formula 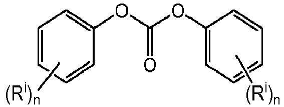 wherein each n is 0 to 2 and each Rⁱ is independently the same or different and is nitro, halo, (C₁₋₁₈hydrocarbyl)carbonyl, or (C₁₋₃alkoxy)carbonyl.
Embodiment 10. The method of embodiment 9, wherein a transesterification selectivity of the catalyst is substantially the same as a transesterification selectivity of a comparable reaction in the presence of a catalyst having the formula M'(OAr')₄, wherein M' is Ti, Zr, or Hf, and is the same as the catalyst, and Ar' is a substituted or unsubstituted C₆₋₂₀ aromatic hydrocarbon group and is the same as the catalyst.
Embodiment 11. The method of embodiment 10, wherein M is Ti and M' is Ti.
Embodiment 12. The method of embodiment 10 or 11, wherein the transesterification selectivity is greater than 98%, preferably greater than 98.5%, more preferably greater than 99%, even more preferably greater than 99.5%.
Embodiment 13: The method of any one or more of embodiment 10 to 12, wherein the diaryl carbonate is bis(4-nitrophenyl)carbonate, bis(2-chlorophenyl)carbonate, bis(4-chlorophenyl)carbonate, bis(methyl salicyl)carbonate, bis(4-methylcarboxylphenyl) carbonate, bis(2-acetylphenyl) carboxylate, or bis(4-acetylphenyl) carboxylate.
Embodiment 14: The method of any one or more of embodiments 10 to 13, wherein the diaryl carbonate is diphenyl carbonate.
Embodiment 15: A method for the manufacture of a polycarbonate, the method comprising manufacturing the diaryl carbonate in accordance with the method of any one or more of embodiments 10 to 14; and reacting the diaryl carbonate with a dihydroxy aromatic compound under conditions effective to provide the polycarbonate.
Embodiment 16: The method of embodiment 15, wherein the dihydroxy aromatic compound is resorcinol, 2,2-bis(4-hydroxyphenyl) propane, 3,3-bis(4-hydroxyphenyl) phthalimidine, 2-phenyl-3,3'-bis(4-hydroxyphenyl) phthalimidine, 3,3-bis(4-hydroxyphenyl)-2-phenylisoindolin-1-one, 1,1-bis(4-hydroxy-3-methylphenyl)cyclohexane, 1,1-bis(4-hydroxyphenyl)-3,3,5-trimethylcyclohexane, or a combination comprising at least one of the foregoing.
Embodiment 17: The method of any one or more of embodiments 15 to 16, wherein a molar ratio of the diaryl carbonate to the dihydroxy aromatic compound is 2:1 to 1:2, preferably 1.5:1 to 1:1.5, more preferably 1.05:1 to 1:1.05, or even more preferably 1:1.
Embodiment 18: A polycarbonate composition, wherein the polycarbonate composition is manufactured by the method of any one or more of embodiments 15 to 17.

The compositions, methods, and articles can alternatively comprise, consist of, or consist essentially of, any appropriate components or steps herein disclosed. The compositions, methods, and articles can additionally, or alternatively, be formulated so as to be devoid, or substantially free, of any steps, components, materials, ingredients, adjuvants, or species that are otherwise not necessary to the achievement of the function or objectives of the compositions, methods, and articles.

The singular forms "a," "an," and "the" include plural referents unless the context clearly dictates otherwise. "Or" means "and/or" unless clearly indicated otherwise by context. The endpoints of all ranges directed to the same component or property are inclusive and independently combinable (e.g., ranges of "less than or equal to 25 wt%, or 5 wt% to 20 wt%," is inclusive of the endpoints and all intermediate values of the ranges of "5 wt% to 25 wt%," etc.). Disclosure of a narrower range or more specific group in addition to a broader range is not a disclaimer of the broader range or larger group.

The term "catalyst," as used herein encompasses the initial starting compound that is used to catalyze a reaction. Thus, the term "catalyst" may or may not correspond to the actual catalytic moiety or active site that effects or is formed during the course of a reaction.

Unless defined otherwise, technical and scientific terms used herein have the same meaning as is commonly understood by one of skill in the art to which this disclosure belongs. A "combination" is inclusive of blends, mixtures, alloys, reaction products, and the like.

As used herein, the term "hydrocarbyl" and "hydrocarbon" refers broadly to a substituent comprising carbon and hydrogen, optionally with 1 to 3 heteroatoms, for example, oxygen, nitrogen, halogen, silicon, sulfur, or a combination thereof; "alkyl" refers to a straight or branched chain, saturated monovalent hydrocarbon group; "alkylene" refers to a straight or branched chain, saturated, divalent hydrocarbon group; "alkenyl" refers to a straight or branched chain monovalent hydrocarbon group having at least two carbons joined by a carbon-carbon double bond; "cycloalkyl" refers to a non-aromatic monovalent monocyclic or multicylic hydrocarbon group having at least three carbon atoms, "cycloalkenyl" refers to a non-aromatic cyclic divalent hydrocarbon group having at least three carbon atoms, with at least one degree of unsaturation; "aryl" refers to an aromatic monovalent group containing only carbon in the aromatic ring or rings; "arylene" refers to an aromatic divalent group containing only carbon in the aromatic ring or rings; "alkylarylene" refers to an aryl group that has been substituted with an alkyl group as defined above, with 4-methylphenyl being an exemplary alkylarylene group; "arylalkylene" refers to an alkyl group that has been substituted with an aryl group as defined above, with benzyl being an exemplary arylalkylene group; "acyl" refers to an alkyl group as defined above with the indicated number of carbon atoms attached through a carbonyl carbon bridge (-C(=O)-); "alkoxy" refers to an alkyl group as defined above with the indicated number of carbon atoms attached through an oxygen bridge (-O-); and "aryloxy" refers to an aryl group as defined above with the indicated number of carbon atoms attached through an oxygen bridge (-O-).

Unless otherwise indicated, each of the foregoing groups can be unsubstituted or substituted, provided that the substitution does not significantly adversely affect synthesis, stability, or use of the compound. The term "substituted" as used herein means that at least one hydrogen on the designated atom or group is replaced with another group, provided that the designated atom's normal valence is not exceeded. When the substituent is oxo (i.e., =O), then two hydrogens on the atom are replaced. Combinations of substituents or variables are permissible provided that the substitutions do not significantly adversely affect synthesis or use of the compound. Exemplary groups that can be present on a "substituted" position include, but are not limited to, cyano; hydroxyl; nitro; alkanoyl (such as a C₂₋₆ alkanoyl group such as acyl); carboxamido; C₁₋₆ or C₁₋₃ alkyl, cycloalkyl, alkenyl, and alkynyl (including groups having at least one unsaturated linkages and from 2 to 8, or 2 to 6 carbon atoms); C₁₋₆ or C₁₋₃ alkoxys; C₆₋₁₀ aryloxy such as phenoxy; C₁₋₆ alkylthio; C₁₋₆ or C₁₋₃ alkylsulfinyl; C₁₋₆ or C₁₋₃ alkylsulfonyl; aminodi(C₁₋₆ or C₁₋₃)alkyl; C₆₋₁₂ aryl having at least one aromatic rings (e.g., phenyl, biphenyl, naphthyl, or the like, each ring either substituted or unsubstituted aromatic); C₇₋₁₉ arylalkylene having 1 to 3 separate or fused rings and from 6 to 18 ring carbon atoms; or arylalkyleneoxy having 1 to 3 separate or fused rings and from 6 to 18 ring carbon atoms, with benzyloxy being an exemplary arylalkyleneoxy. The number of carbon atoms indicated for a group is exclusive of any substituents.

All cited patents, patent applications, and other references are incorporated herein by reference in their entirety. However, if a term in the present application contradicts or conflicts with a term in the incorporated reference, the term from the present application takes precedence over the conflicting term from the incorporated reference.

While typical embodiments have been set forth for the purpose of illustration, the foregoing descriptions should not be deemed to be a limitation on the scope herein. Accordingly, various modifications, adaptations, and alternatives can occur to one skilled in the art without departing from the spirit and scope herein.

## Claims

1. A catalyst comprising a compound of the formula
[(R)ₘCp]ₙM(OAr)₂L₂₋ₙ
wherein
each Cp is cyclopentadienyl,
each R is independently the same or different, and is a substituted or unsubstituted C₁₋₆ alkyl group,
M is Ti, Zr, or Hf,
each Ar is independently the same or different, and is a substituted or unsubstituted C₆₋₂₀ aromatic hydrocarbon group,
each L is independently the same or different, and is a halogen or a substituted or unsubstituted C₁₋₆ alkoxide group,
m is 0 to 5, and
n is 1 or 2.

2. The catalyst of claim 1, wherein the compound comprises the formula 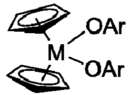 wherein M is Ti, Zr, or Hf, preferably wherein M is Ti.

3. A method for the synthesis of the catalyst of claim 1 or 2, the method comprising:
reacting a metallocene halide of the formula
[(R)ₘCp]ₙMX₄₋ₙ
wherein X is F, Cl, Br, or I, with an aromatic hydroxy compound of the formula
ArOH
to obtain the catalyst.

4. The method of claim 3, wherein the metallocene halide has the formula

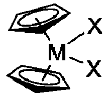

5. The method of claim 4 or 5, wherein M is Ti and X is Cl.

6. The method of any one or more of claims 4 to 6, wherein the reacting comprises combining the metallocene halide, the aromatic hydroxy compound, and a base.

7. The method of claim 7, wherein
a molar ratio of the phenol to the metallocene is 1:1 to 6:1, preferably 2:1 to 4:1, more preferably 2:1 to 3:1;
the base is pyridine, N,N'-dimethylamino, N-methylpyrrolidone, dimethylamine, trimethyl amine, triethyl amine, ethyldiisopropyl amine, dimethylformamide, N-methylformamide, N-methylimidazole, N,N-dimethylaniline, pyridine, ammonia, or a combination comprising at least one of the foregoing; and
the reacting is at a temperature of 25 °C to 200 °C, preferably 25 °C to 180 °C, more preferably 25 °C to 150 °C, for a time of 10 minutes to 6 hours, preferably 20 minutes to 4 hours, more preferably 30 minutes to 3 hours.

8. A method for the synthesis of a diaryl carbonate, the method comprising
reacting, in the presence of the catalyst of any one or more of claims 1 to 3, or made by the methods of any one or more of claims 4 to 8
a dialkyl carbonate of the formula
O=C(OZ)₂
wherein each Z is independently the same or different, and is a C₁₋₄ alkyl group, with
an aromatic aromatic alcohol of the formula 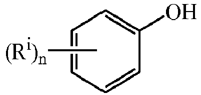
wherein n is 0 to 2 and each Rⁱ is independently the same or different and is nitro, halo, (C₁₋₁₈hydrocarbyl)carbonyl, or (C₁₋₃alkoxy)carbonyl, to provide the diaryl carbonate of the formula 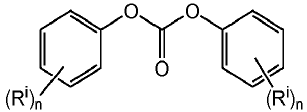 wherein each n is 0 to 2 and each Rⁱ is independently the same or different and is nitro, halo, (C₁₋₁₈hydrocarbyl)carbonyl, or (C₁₋₃alkoxy)carbonyl.

9. The method of claim 9, wherein a transesterification selectivity of the catalyst is substantially the same as a transesterification selectivity of a comparable reaction in the presence of a catalyst having the formula
M'(OAr')₄,
wherein
M' is Ti, Zr, or Hf, and is the same as the catalyst, and
Ar' is a substituted or unsubstituted C₆₋₂₀ aromatic hydrocarbon group and is the same as the catalyst.

10. The method of claim 10, wherein M is Ti and M' is Ti.

11. The method of claim 10 or 11, wherein the transesterification selectivity is greater than 98%, preferably greater than 98.5%, more preferably greater than 99%, even more preferably greater than 99.5%.

12. The method of any one or more of claim 10 to 13, wherein
the diaryl carbonate is diphenyl carbonate bis(4-nitrophenyl)carbonate, bis(2-chlorophenyl)carbonate, bis(4-chlorophenyl)carbonate, bis(methyl salicylate)carbonate, bis(4-methylcarboxyphenyl) carbonate, bis(2-acetylphenyl) carboxylate, or bis(4-acetylphenyl) carboxylate;
preferably wherein the diaryl carbonate is diphenyl carbonate.

13. A method for the manufacture of a polycarbonate, the method comprising
manufacturing the diaryl carbonate in accordance with the method of any one or more of claims 8 to 12; and
reacting the diaryl carbonate with a dihydroxy aromatic reactant under conditions effective to provide the polycarbonate.

14. The method of claim 13, wherein the dihydroxy aromatic compound is resorcinol, 2,2-bis(4-hydroxyphenyl) propane, 3,3-bis(4-hydroxyphenyl) phthalimidine, 2-phenyl-3,3'-bis(4-hydroxyphenyl) phthalimidine, 3,3-bis(4-hydroxyphenyl)-2-phenylisoindolin-1-one, 1,1-bis(4-hydroxy-3-methylphenyl)cyclohexane, 1,1-bis(4-hydroxyphenyl)-3,3,5-trimethylcyclohexane, or a combination comprising at least one of the foregoing.

15. A polycarbonate composition manufactured by the method of claims 13 or 14.
